# EUROPÄISCHE PATENTSCHRIFT

(11) **EP 3 416 700 B1**
(45) Veröffentlichungstag und Bekanntmachung des Hinweises auf die Patenterteilung: **22.01.2020**
(21) Anmeldenummer: 17704668.7
(22) Anmeldetag: 09.02.2017
(51) Int. Cl.: A61M 1/34, A61M 5/44, A61M 1/36

(54) **VORRICHTUNG ZUR DURCHFÜHRUNG EINER EXTRAKORPORALEN BLUTBEHANDLUNG**
DEVICE FOR CARRYING OUT AN EXTRA-CORPOREAL TREATMENT OF BLOOD
DISPOSITIF POUR RÉALISER UN TRAITEMENT EXTRA-CORPOREL DU SANG

(30) Priorität: 16.02.2016 DE 102016001765
(43) Veröffentlichungstag der Anmeldung: 26.12.2018
(73) Patentinhaber: Fresenius Medical Care Deutschland GmbH, 61352 Bad Homburg (DE)
(72) Erfinder: BREUEL, Lars, 99310 Witzleben/OT Achelstädt (DE); MAGER, Gerhard, 61352 Bad Homburg (DE); VERCH, Gerhard, 65191 Wiesbaden (DE); KLEWINGHAUS, Juergen, 61440 Oberursel (DE)
(74) Vertreter: Herrmann, Uwe
(86) Internationale Anmeldenummer: PCT/EP2017/000184
(87) Internationale Veröffentlichungsnummer: WO 2017/140416

(56) Entgegenhaltungen:
- EP-A1- 0 763 367
- EP-A1- 2 291 207
- EP-A2- 2 324 871
- WO-A1-02/062454
- CA-A1- 2 704 411
- DE-A1-102009 008 346
- DE-A1-102014 008 546
- DE-A1-102014 108 444
- DE-B3-102006 042 120
- US-A1- 2008 243 045
- US-B1- 6 824 524

## Beschreibung

Die Erfindung betrifft eine Vorrichtung zur Durchführung einer extrakorporalen Blutbehandlung, in der dem Patienten eine Substitutionsflüssigkeit verabreicht wird.

In der Plasmapherese wird anhand eines in einem extrakorporalen Blutkreislauf angeordneten Filters Plasma aus dem Blut eines Patienten abgetrennt. Das Verfahren wird beispielsweise zur Behandlung mancher Autoimmunerkrankungen verwendet. Bei einer Variante der Methode, die als Plasmaaustausch bezeichnet wird, wird das entfernte Plasma verworfen und eine Substitutionsflüssigkeit in den extrakorporalen Blutkreislauf gegeben.

Die Erfindung ist aber nicht auf die Plasmapherese beschränkt sondern allgemein auf Geräte zur extrakorporalen Blutbehandlung anwendbar, in denen dem Patienten eine nicht körpereigene Substitutionsflüssigkeit zugeführt wird.

In der Substitutionsleitung ist für gewöhnlich wenigstens ein Heizbeutel angeordnet, in dem die Substitutionsflüssigkeit auf Körpertemperatur erwärmt wird. Am Behandlungsende wird die in den Heizbeuteln enthaltene Substitutionsflüssigkeit gemäß dem Stand der Technik anhand einer Verdrängung durch eine Verdrängungsflüssigkeit in den extrakorporalen Blutkreislauf infundiert. Dabei kann es in der Substitutionsleitung und insbesondere in den Heizbeuteln zu einer Vermischung der beiden Flüssigkeiten kommen, was eine vollständige Reinfusion der Substitutionslösung verhindert. Sofern als Substitutionsflüssigkeit Plasma und als Verdrängungsflüssigkeit eine physiologische NaCI-Lösung verwendet wird, begünstigt auch die größere Dichte des Plasmas (1,028 gegenüber 1,0046 g/ml) in den zur Entlüftung normalerweise von unten nach oben befüllten Heizbeuteln die Vermischung beider Flüssigkeiten.

Beispielsweise ist aus der CA 2704411 A1 eine Vorrichtung und ein Verfahren zum Ausgleichen von Strömen von Nierenersatzfluid wird offenbart. Das Verfahren verwendet Drucksteuerungen und Druckerfassungsvorrichtungen, um den Fluss von frischem Dialysat und verbrauchtem Dialysat genauer zu messen und auszugleichen.

Zudem betrifft die Druckschrift DE 102014 008546 A1 ein Haemofiltrationsgerät mit einem Haemofilter mit einer Zuleitung zum Zuführen eines Blutstromes von einem Patienten zu dem Haemofilter sowie mit einer Rückführleitung zum Zurückführen des Blutstromes zu dem Patienten.

Zudem offenbart die Druckschrift US 6824524 B1 einen Schlauch mit einem extrakorporalen Zirkulationskanal mit offener Schleife, der aus zwei Teilen besteht, wobei ein Teil das zu reinigende Blut extrahiert und der andere das gereinigte Blut zurückführt.

Die Druckschrift US2008/243045 A1 offenbart eine Vorrichtung und ein Verfahren zur gleichzeitigen extrarenalen Blutreinigungstherapie und Atmungsunterstützungstherapie.

Zudem offenbart die Druckschrift EP 2324871 A2 ein maschinenlesbares Medium, das Anweisungen enthält, die von einem oder mehreren Prozessoren ausgeführt werden können, wobei die Anweisungen Anweisungen zum Pumpen von Blut aus einem Blutstrom eines Patienten in eine Zugangsleitung umfassen, Anweisungen zum Einführen einer Antikoagulanslösung in das gepumpte Blut, Anweisungen zum Filtern des gepumpten Blut, Anweisungen zur Abgabe des gepumpten Blutes aus dem Filterschritt an eine Rückführleitung, Anweisungen zum Einführen von mindestens einer Substitutionsflüssigkeit in das gepumpte Blut, Anweisungen zum Einleiten einer Calciumlösung in das durch die Rückführleitung strömende Blut und Anweisungen zur Rückführung des Bluts Blut zurück in den Blutkreislauf des Patienten.

Aufgabe der Erfindung ist es, eine gattungsgemäße Vorrichtung bereitzustellen, in der diese Nachteile vermieden werden.

Die Erfindung wird durch die Merkmale des unabhängigen Anspruchs 1 definiert. Vor diesem Hintergrund betrifft die Erfindung eine Vorrichtung zur Durchführung einer extrakorporalen Blutbehandlung, in der dem Patienten eine Substitutionsflüssigkeit verabreicht wird, wobei die Vorrichtung einen extrakorporalen Blutkreislauf und eine in den extrakorporalen Blutkreislauf mündende Substitutionsleitung umfasst und wobei die Substitutionsleitung wenigstens ein Heizbehältnis aufweist. Erfindungsgemäß ist vorgesehen, dass stromabwärts des oder der Heizbehältnisse eine Pumpe zur Förderung von Substitutionsflüssigkeit in den extrakorporalen Blutkreislauf in der Substitutionsleitung angeordnet ist.

Durch die Anordnung der Pumpe stromabwärts der vergleichsweise großvolumigen Heizbehältnisse können diese in der Endphase der Behandlung nach einer Unterbrechung der Zufuhr von Flüssigkeit auf der Vorlaufseite leergesaugt werden. In einer Ausführungsform sind die Heizbehältnisse kompressibel. Vorzugsweise handelt es sich um Kunststoffbeutel. In diesem Fall kann sich das Beutelvolumen mit dem sinkenden enthaltenen Flüssigkeitsvolumen beim Leersaugen durch die Pumpe verkleinern.

In einer Ausführungsform ist stromaufwärts des oder der Heizbehältnisse in der Substitutionsleitung eine Klemme angeordnet, die im geschlossenen Zustand den Fluss von Substitutionslösung unterbindet. Eine Möglichkeit der Unterbrechung der Zufuhr von Flüssigkeit auf der Vorlaufseite liegt im Vorsehen einer Klemme.

In einer Ausführungsform weist die Vorrichtung ferner eine Steuereinheit auf, die mit der Pumpe und gegebenenfalls der Klemme in Verbindung steht und derart ausgebildet ist, dass in einem Betriebsmodus der Vorrichtung die Klemme geschlossen und die Pumpe betrieben wird. Dieser Betriebsmodus entspricht der Endphase der Behandlung, wenn keine zusätzliche Substitutionsflüssigkeit mehr benötigt wird.

Eine Ausführungsvariante umfasst dabei eine manuelle Klemme, die nicht mit der Steuereinheit in Verbindung steht. Wenn die Steuereinheit nur mit der Pumpe in Verbindung steht, dann kann der Zustand der Klemme beispielsweise anhand eines Interfaces durch den Anwender an die Steuereinheit übermittelt werden.

In einer Ausführungsform ist stromaufwärts des oder der Heizbehältnisse, zwischen dem oder den Heizbehältnissen und der Pumpe sowie stromabwärts der Pumpe jeweils mindestens eine Schnittstelle angeordnet, an der die Substitutionsleitung getrennt werden kann, wobei die Schnittstellen derart miteinander kompatibel ausgebildet sind, dass das oder die Heizbehältnisse alternativ auch stromabwärts der Pumpe eingesetzt werden könnten. Die Konfiguration mit der Pumpe stromabwärts des oder der Behältnisse muss nicht der Konfiguration der Vorrichtung entsprechen, die in der Normalphase der Behandlung vorliegt. Ebenso ist denkbar, die Konfiguration vor Einleitung der Endphase der Behandlung zu ändern und dabei insbesondere die Lage der Pumpe relativ zu dem oder den Behältnissen zu ändern. Wenn stromaufwärts und stromabwärts der Pumpe jeweils eine Schnittstelle angeordnet ist, an der die Substitutionsleitung getrennt werden kann, können die Behältnisse wahlweise stromaufwärts oder stromabwärts der Pumpe in die Substitutionsleitung integriert werden. So ist denkbar, dass die Vorrichtung während der Behandlung eine Normalkonfiguration einnimmt, in der die Pumpe stromaufwärts der Behältnisse angeordnet ist, und in der Endphase der Behandlung die Konfiguration mit der stromabwärtigen Pumpe einnimmt, in der die Pumpe stromabwärts der Behältnisse angeordnet ist. Die Umstellung kann dabei maschinell oder manuell erfolgen.

Es ist eine weitere Pumpe zur Förderung von Substitutionsflüssigkeit in den extrakorporalen Blutkreislauf stromaufwärts des oder der Heizbehältnisse in der Substitutionsleitung angeordnet. Eine Möglichkeit der Unterbrechung der Zufuhr von Flüssigkeit auf der Vorlaufseite liegt auch im Vorsehen einer weiteren und stromaufwärts des oder der Behältnisse angeordneten Pumpe, die während der Endphase der Behandlung außer Betrieb gesetzt wird und in diesem Zustand eine Sperrwirkung entfaltet. Gleichzeitig kann diese stromaufwärtige Pumpe natürlich auch der Fluidförderung beispielsweise während der Normalphase der Behandlung dienen.

In einer Ausführungsform weist die Substitutionsleitung im Bereich der stromabwärtigen Pumpe eine Bypassleitung auf, in der vorzugsweise ein Rückschlagventil angeordnet ist, das einen Fluss nur in Richtung des extrakorporalen Blutkreislaufs zulässt. Soweit vorgesehen ist, die stromabwärtige Pumpe während der Normalphase der Behandlung nicht zu betreiben und stattdessen die stromaufwärtige Pumpe zu verwenden, muss eine Bypassleitung vorhanden sein, damit die Substitutionsflüssigkeit an der stillstehenden und insoweit eine Sperrwirkung entfaltenden stromabwärtigen Pumpe vorbeifließen kann. Das Rückschlagventil dient dazu, in der Endphase der Behandlung, wenn die stromaufwärtige Pumpe stillsteht und die stromabwärtige Pumpe zum Leersaugen des oder der Heizbehältnisse betrieben wird, einen Kurzschluss durch den Bypass zu vermeiden.

Die Vorrichtung weist ferner eine Steuereinheit auf, die mit den Pumpen in Verbindung steht und derart ausgebildet ist, dass in einem Betriebsmodus der Vorrichtung die stromaufwärtige Pumpe gestoppt und die stromabwärtige Pumpe betrieben wird. Dieser Betriebsmodus entspricht der Endphase der Behandlung, wenn keine zusätzliche Substitutionsflüssigkeit mehr benötigt wird.

Vor dem eingangs genannten Hintergrund betrifft die Erfindung ferner eine Vorrichtung zur Durchführung einer extrakorporalen Blutbehandlung, in der dem Patienten eine Substitutionsflüssigkeit verabreicht wird, wobei die Vorrichtung einen extrakorporalen Blutkreislauf und eine in den extrakorporalen Blutkreislauf mündende Substitutionsleitung umfasst und wobei die Substitutionsleitung wenigstens eine Pumpe zur Förderung von Substitutionsflüssigkeit in den extrakorporalen Blutkreislauf und wenigstens ein Heizbehältnis aufweist. Erfindungsgemäß ist hierbei vorgesehen, dass das stromaufwärtige Ende der Substitutionsleitung mit einem Sterilfilter versehen ist und in einen gasgefüllten Raum ragt. Vorzugsweise ragt das stromaufwärtige Ende der Substitutionsleitung schlicht in die Umgebungsluft, die vor Eintritt in die Substitutionsleitung am Sterilfilter sterilisiert wird.

Dies entspricht der Konfiguration der gattungsgemäßen Vorrichtung während der Endphase der Behandlung, in der der in den Heizbehältnissen vorhandene Rest an Substitutionsflüssigkeit in den extrakorporalen Blutkreislauf infundiert werden soll, in der jedoch keine neue Substitutionsflüssigkeit und keine Verdrängungsflüssigkeit in die Substitutionsleitung nachlaufen sollen, sondern nur Gas bzw. Luft.

In einer Ausführungsform dieser Erfindungsvariante ist die Pumpe stromabwärts der Heizbehältnisse angeordnet. In einer anderen Ausführungsform dieser Erfindungsvariante sind die Heizbehältnisse so angeordnet, dass die von oben nach unten durchströmt werden. Diese Ausführungsformen stellen eine verbesserte Funktion des Systems sicher.

In einer Ausführungsform weist die Vorrichtung ferner eine Steuereinheit und gegebenenfalls einen Sensor zur Messung der Fördermenge der Pumpe auf, wobei die Steuereinheit mit der Pumpe und gegebenenfalls mit dem Mengensensor in Verbindung steht und so ausgebildet ist, dass in einer Betriebsphase der Vorrichtung die Fördermenge der Pumpe gemessen und die Pumpe bei Erreichen einer bestimmten Gesamtfördermenge abgeschaltet wird.

In einer Ausführungsform weisen der extrakorporale Blutkreislauf stromabwärts der Mündungsstelle der Substitutionsleitung eine Tropfkammer auf und die Vorrichtung ferner eine Steuereinheit und einen Sensor zur Messung des Füllstands der Tropfkammer auf, wobei die Steuereinheit mit der Pumpe und mit dem Füllstandsensor in Verbindung steht und so ausgebildet ist, dass in einer Betriebsphase der Füllstand der Tropfkammer gemessen und die Pumpe bei Erreichen eines bestimmten Mindestfüllstands abgeschaltet wird.

Diese Betriebsphasen entsprechen jeweils der Endphase der Behandlung, wenn keine zusätzliche Substitutionsflüssigkeit mehr benötigt wird.

Vorzugsweise dient die erfindungsgemäße Vorrichtung der Durchführung einer Plasmapheresebehandlung. Bei der oder den Pumpen handelt es sich vorzugsweise um peristaltische Pumpen.

Vor dem eingangs genannten Hintergrund wird ferner ein Verfahren zur Durchführung einer extrakorporalen Blutbehandlung und vorzugsweise einer Plasmapheresebehandlung angedacht, bei dem in einer Endphase der Behandlung die Zufuhr von Flüssigkeit stromaufwärts der Substitutionsleitung unterbrochen wird und der oder die Heizbeutel, in denen noch Substitutionsflüssigkeit vorhanden ist, durch eine stromabwärts dieser Beutel angeordnete Pumpe, welche die Substitutionsflüssigkeit in Richtung des extrakorporalen Blutkreislaufs fördert, leergesaugt wird. Vorzugsweise erfolgt dieses Verfahren unter Verwendung einer Vorrichtung, die in der Endphase der Behandlung die in einem der Ansprüche 1 bis 7 beschriebene Konfiguration hat.

Des Weiteren wird ein Verfahren zur Durchführung einer extrakorporalen Blutbehandlung und vorzugsweise einer Plasmapheresebehandlung angedacht, bei dem in einer Endphase der Behandlung Gas und insbesondere Luft eingesetzt wird, um die in dem oder den Heizbeuteln der Substitutionsleitung enthaltene Substitutionsflüssigkeit in Richtung des extrakorporalen Blutkreislaufs zu verdrängen. Vorzugsweise erfolgt dieses Verfahren unter Verwendung einer Vorrichtung, die in der Endphase der Behandlung die in einem der Ansprüche 8 bis 10 beschriebene Konfiguration hat.

Allen erfindungsgemäßen Vorrichtungen und angedachten Verfahren ist gemein, dass keine Verdrängung der Substitutionsflüssigkeit mit einer Verdrängungsflüssigkeit wie beispielsweise physiologischer Kochsalzlösung erfolgt. Vielmehr wird entweder die Substitutionsleitung stromaufwärts der Heizbehältnisse unterbrochen und die Heizbehältnisse werden leergesaugt, oder eine Verdrängung findet mit Gas und insbesondere Luft statt.

Weitere Einzelheiten und Vorteile der Erfindung ergeben sich aus den nachfolgend anhand der Figuren erklärten Ausführungsbeispielen. In den Figuren zeigen:
- Figur 1:: ein Flussbild einer Vorrichtung für den Plasmaaustausch während der Normalphase der Behandlung;
- Figur 2:: ein Flussbild einer Vorrichtung für den Plasmaaustausch während der Endphase der Behandlung, wobei eine Verdrängung der Substitutionsflüssigkeit mit einer Verdrängungsflüssigkeit erfolgt;
- Figur 3:: ein Flussbild einer erfindungsgemäßen Vorrichtung für den Plasmaaustausch während der Endphase der Behandlung, wobei eine Verdrängung der Substitutionsflüssigkeit mit Luft erfolgt;
- Figur 4:: ein Flussbild einer Variante erfindungsgemäßen Vorrichtung für den Plasmaaustausch, wobei während der Endphase der Behandlung die Heizbehältnisse leergesaugt werden; und
- Figur 5:: ein Flussbild einer weiteren Variante einer erfindungsgemäßen Vorrichtung für den Plasmaaustausch, wobei während der Endphase der Behandlung die Heizbehältnisse leergesaugt werden.

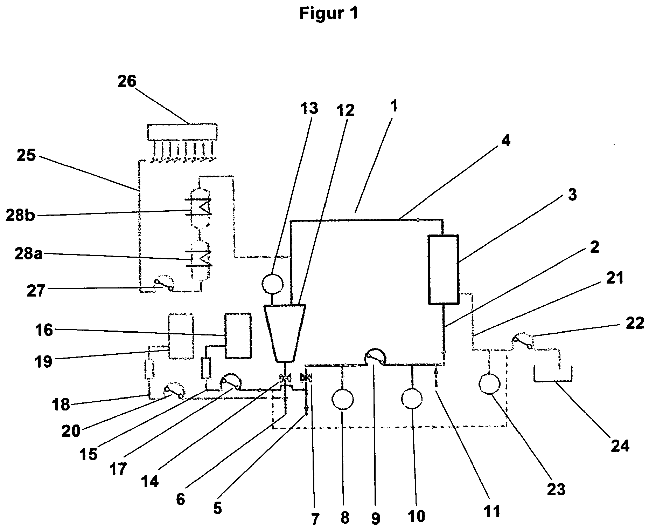

Figur 1 zeigt eine gattungsgemäße Vorrichtung während der Normalphase der Behandlung, in der kontinuierlich frische Substitutionsflüssigkeit aus einem Reservoir bezogen und an den Patienten verabreicht wird.

Der extrakorporale Blutkreislauf der Vorrichtung ist mit dem Bezugszeichen 1 gekennzeichnet. Er umfasst eine arterielle Leitung 2, einen Plasmafilter 3 und eine venöse Leitung 4. An der Vorlaufseite der arteriellen Leitung 2 ist ein arterieller Port 5 und an der Rücklaufseite der venösen Leitung 4 ein venöser Port 6 angeordnet. Diese Ports 5 und 6, bei denen es sich beispielsweise um Nadeln handeln kann, dienen zum Anschluss der Vorrichtung an einen Patienten.

In der arteriellen Leitung 2 sind in Flussrichtung gestaffelt eine arterielle Klemme 7, ein Drucksensor 8 zur Messung des arteriellen Drucks, eine Blutpumpe 9, ein weiterer Drucksensor 10 zur Messung des Pumpdrucks und eine Leitung 11 zur Zufuhr eines Antikoagulationsmittels wie beispielsweise Heparin angeordnet.

In der venösen Leitung 4 befinden sich eine Tropfkammer 12, ein Drucksensor 13 zur Messung des venösen Drucks und eine venöse Klemme 14.

Zusätzlich ist an die arterielle Leitung 2 zwischen dem arteriellen Port 5 und der arteriellen Klemme 7 ein Zudosiersystem 15 für Citrat angeschlossen, das ein Citratreservoir 16 und eine Citratpumpe 17 umfasst. An die venöse Leitung 4 zwischen der venösen Klemme 14 und dem venösen Port 6 ein Zudosiersystem 18 für Calciumionen angeschlossen, das ein Calciumionenreservoir 19 und eine Calciumpumpe 20 umfasst.

Der Plasmafilter 3 umfasst eine semipermeable Membran, die den extrakorporalen Blutkreislauf 1 von einem Abflusssystem 21 für aus dem Blut abgetrenntes Plasma trennt. Das Abflusssystem 21 umfasst eine Filtrationspumpe 22, einen Drucksensor 23 zur Messung des Filtrationsdrucks sowie einen Abfluss 24 zum Sammeln bzw. Entsorgen des abgetrennten Plasmas.

Ferner umfasst die Vorrichtung eine Substitutionsleitung 25, die stromaufwärts der Tropfkammer 12 in die venöse Leitung 4 des extrakorporalen Blutkreislaufs 1 mündet. Vorlaufseitig der Substitutionsleitung 25 ist ein Reservoir 26 für Substitutionsflüssigkeit angeordnet. In der Substitutionsleitung 25 ist ferner eine Substitutionspumpe 27 angeordnet, die der Förderung von Substitutionsflüssigkeit aus dem Reservoir 26 in die venöse Leitung 4 dient. Des Weiteren umfasst die Substitutionsleitung 25 noch Heizbeutel 28a und 28b, die einen Wärmetauscher umfassen und dazu dienen, die Temperatur der Substitutionsflüssigkeit auf Körpertemperatur anzuheben.

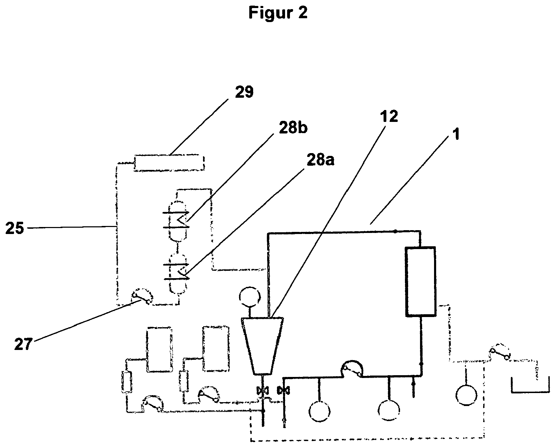

Figur 2 zeigt dieselbe Vorrichtung während der Endphase der Behandlung, in der keine frische Substitutionsflüssigkeit mehr bezogen und an den Patienten verabreicht wird. Stattdessen soll der noch in der Substitutionsleitung 25 und insbesondere in den Heizbeuteln 28a und 28b befindliche Rest der Substitutionsflüssigkeit durch Verdrängung mit einer Verdrängungsflüssigkeit in den extrakorporalen Blutkreislauf 1 gefördert werden. Zu diesem Zweck wird in der Endphase der Behandlung das Reservoir 26 für Substitutionsflüssigkeit durch ein Reservoir 29 für eine Verdrängungsflüssigkeit ersetzt. Die Substitutionspumpe 27 fördert dann so lange Verdrängungsflüssigkeit aus dem Reservoir 29, bis das gesamte Volumen der Substitutionsleitung 25 und der Heizbeutel 28a und 28b leergespült ist. Die Bestimmung des geförderten Volumens kann beispielsweise anhand eines in den Figuren nicht dargestellten Flusssensors erfolgen. Bei dieser Methode ergeben sich jedoch die im einleitenden Teil der Beschreibung genannten Probleme einer Vermischung und unvollständigen Verdrängung.

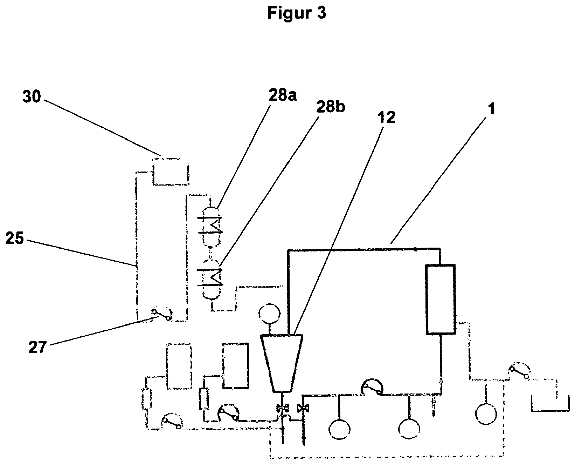

In Figur 3 ist eine erfindungsgemäß ausgebildete Vorrichtung während der Endphase der Behandlung dargestellt.

Anstelle der physiologischen NaCI-Lösung (vgl. Reservoir 29 der Figur 2) wird an das Schlauchsystem nun kein Flüssigkeitsreservoir sondern lediglich ein Sterilfilter 30 angeschlossen. Da die Heizbeutel 28a und 28b von unten nach oben durchflossen werden, sollten sie in dieser Lösung (was in der Figur nicht dargestellt ist) aus der Heizung entfernt und umgedreht werden, beispielsweise umgekehrt an eine Infusionsstange gehängt werden.

Anschließend wird mit der Substitutionspumpe 27 so lange Luft gefördert, bis das Plasma in den Heizbeuteln 28a und 28b sowie dem Schlauchsystem 25 bis zur venösen Tropfkammer 12 vollständig reinfundiert ist. Die Reinfusion ist vom Personal gut zu überwachen, da die Grenzfläche zwischen Luft und Plasma gut sichtbar ist.

Eine Gefährdung geht davon nicht aus, da eventuell geförderte Luft in der venösen Tropfkammer 12 abgefangen und bei großen Mengen dort oder in einem nachfolgenden und nicht in der Figur dargestellten Detektor sicher erkannt werden könnte. Die Reinfusion könnte automatisiert werden, in dem das Ende der Reinfusion durch das von der Substitutionspumpe 27 zu fördernde Volumen oder durch den Abfall des Pegels in der zur venösen Tropfkammer 12 bestimmt wird.

Vorteile dieser Variante umfassen eine nahezu vollständige Plasma-Reinfusion, wobei die Kosten für die physiologische NaCI-Reinfusionslösung entfallen.

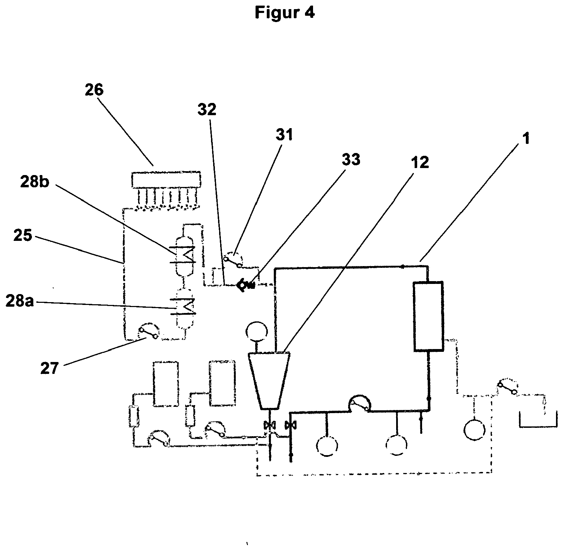

In Figur 4 ist eine weitere erfindungsgemäße Variante einer gattungsgemäßen Vorrichtung während der Endphase der Behandlung dargestellt.

Hier erfolgt die Reinfusion des in den Heizbeuteln 28a und 28b sowie in der Leitung 25 enthaltenen Plasmas durch eine zusätzliche Pumpe 31, die parallel zu einer Bypassleitung 32 in der Substitutionsleitung 25 zwischen den Heizbeuteln 28a und 28b und dem extrakorporalen Blutkreislauf 1 geschaltet ist. Damit die Heizbeutel 28a und 28b von dieser zusätzlichen Pumpe 31 leergesaugt werden können, ist in der Bypassleitung ein Rückschlagventil 33 angeordnet.

Während der Reinfusion (Endphase der Behandlung) wird die Substitutionspumpe 27 gestoppt und die zusätzliche Pumpe 31 fördert das in den Heizbeuteln 28a und 28b befindliche Plasma zum Blutschlauch 1. Die Reinfusion kann automatisiert werden, indem das Ende der Reinfusion durch das von der zusätzlichen Pumpe 31 zu fördernde Volumen bestimmt wird.

In einer Variante könnte durch Anschließen der Substitutionsleitung 25 mit der zusätzlichen Pumpe 31 an den venösen Blasenfänger 12 ein in manchen Geräten ohnehin an der Plasmainfusionsstelle vorhandenes Rückschlagventil genutzt werden.

Vorteile dieser Lösung umfassen eine nahezu vollständige Plasma-Reinfusion, wobei die Kosten für die physiologische NaCI-Reinfusionslösung entfallen. Ferner ist ein automatisiertes Handling der Reinfusion möglich. Eine Gefährdung für den Patienten besteht aus den bereits im Zusammenhang mit der Diskussion der Figur 3 genannten Gründen nicht.

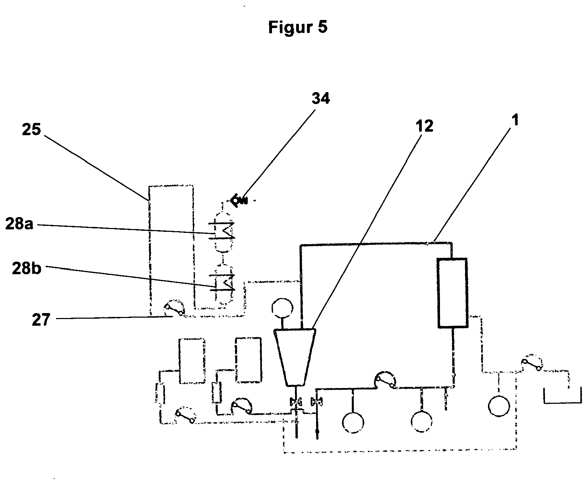

In Figur 5 ist eine weitere erfindungsgemäße Variante einer gattungsgemäßen Vorrichtung während der Endphase der Behandlung dargestellt.

Gemäß dieser Variante für eine nahezu vollständige Plasma-Reinfusion ist ein Umklemmen der Heizbeutels 28a und 28b vorgesehen. Vor Beginn der Endphase der Behandlung werden die Heizbeutel 28a und 28b aus der Substitutionsleitung 25 entfernt und vor der Substitutionspumpe 27 angeschlossen. Die andere Seite der Heizbeutel 28a und 28b kann verschlossen werden, beispielsweise anhand einer Klemme 34. Die Substitutionsleitung 25 wird an der Stelle wieder geschlossen, an der die Heizbeutel 28a und 28b waren. Nun kann durch die Substitutionspumpe 27 das in den Heizbeuteln 28a und 28b befindliche Plasma zum extrakorporalen Blutkreislauf 1 gefördert werden.

Vorteile dieser Variante umfassen eine nahezu vollständige Plasma-Reinfusion, wobei die Kosten für eine physiologische NaCI-Reinfusionslösung entfallen.

Eine weitere erfindungsgemäße Variante wird ebenfalls anhand der Figur 5 diskutiert. Gemäß dieser Variante kann eine Reinfusion durch Verschließen der Anschlussleitung zum Reservoir 26 (dargestellt in Figur 1) erfolgen.

Diese Lösung setzt das ständige Umkehren der Anordnung Heizbeutel 28a und 28b im Verhältnis zur Substitutionspumpe 27 voraus, das heißt die Anordnung der Substitutionspumpe 27 stromabwärts der Heizbeutel 28a und 28b, wie dies in der Figur 5 gezeigt ist. In dieser Variante kann während der Normalphase der Behandlung erst das Plasma in den Heizbeuteln 28a und 28b vorgewärmt und danach durch Substitutionspumpe 27 in den extrakorporalen Blutkreislauf 1 gefördert werden.

Befinden sich die Heizbeutel 28a und 28b stromaufwärts der Substitutionspumpe 27, so kann zur Reinfusion der Zugang zum Reservoir 26 beispielsweise manuell verschlossen werden. Dies reicht aus um anhand der Substitutionspumpe 27 die Heizbeutel 28a und 28b zu entleeren, ohne eine zusätzliche Pumpe vorsehen zu müssen. Voraussetzung ist eine Möglichkeit, den Zugang zum Reservoir 26 manuell oder automatisiert durch eine Maschinenvorrichtung wie beispielsweise eine Klemme 34 zu verschließen. Nach dem Verschließen kann ein vordefiniertes Volumen durch die Pumpe 3 gefördert werden, das sich aus dem rechnerischen Füllvolumen der Heizbeutel 28a und 28b ergibt.

Vorteile dieser Variante umfassen eine nahezu vollständige Plasma-Reinfusion, eine Einsparung der Kosten für die physiologische NaCI-Reinfusionslösung und keine Kosten für zusätzliche Schlauchkupplungen oder andere zusätzlichen Teile.

Bei jeder der genannten erfindungsgemäßen Varianten ist es möglich, die Steuereinheit der Vorrichtung so auszubilden, dass während der Endphase der Behandlung weiterhin Plasma über den Plasmafilter 3 abgezogen werden soll (Nullbilanz) oder ob dieser Abzug stoppen soll (Bolusgabe).

## Patentansprüche

1. Vorrichtung zur Durchführung einer extrakorporalen Blutbehandlung, in der dem Patienten eine Substitutionsflüssigkeit verabreicht wird, wobei die Vorrichtung einen extrakorporalen Blutkreislauf (1) und eine in den extrakorporalen Blutkreislauf (1) mündende Substitutionsleitung (25) umfasst und wobei die Substitutionsleitung (25) wenigstens ein Heizbehältnis (28) aufweist, wobei stromabwärts des oder der Heizbehältnisse (28) eine Pumpe (31) zur Förderung von Substitutionsflüssigkeit in den extrakorporalen Blutkreislauf (1) in der Substitutionsleitung (25) angeordnet ist und **gekennzeichnet dadurch, dass** eine weitere Pumpe (27) zur Förderung von Substitutionsflüssigkeit in den extrakorporalen Blutkreislauf (1) stromaufwärts des oder der Heizbehältnisse (28) in der Substitutionsleitung (25) angeordnet ist und die Vorrichtung ferner eine Steuereinheit aufweist, die mit den Pumpen (27, 31) in Verbindung steht und derart ausgebildet ist, dass in einem Betriebsmodus der Vorrichtung die stromaufwärtige Pumpe (27) gestoppt und die stromabwärtige Pumpe (31) betrieben wird.

2. Vorrichtung nach Anspruch 1, **dadurch gekennzeichnet, dass** stromaufwärts des oder der Heizbehältnisse in der Substitutionsleitung eine Klemme (34) angeordnet ist, die im geschlossenen Zustand den Fluss von Substitutionslösung unterbindet.

3. Vorrichtung nach Anspruch 2, **dadurch gekennzeichnet, dass** die Vorrichtung ferner eine Steuereinheit aufweist, die mit der Pumpe (27) und gegebenenfalls ferner der Klemme (34) in Verbindung steht und derart ausgebildet ist, dass in einem Betriebsmodus der Vorrichtung die Klemme geschlossen und die Pumpe (27) betrieben wird.

4. Vorrichtung nach einem der vorhergehenden Ansprüche, **dadurch gekennzeichnet, dass** stromaufwärts des oder der Heizbehältnisse (28), zwischen dem oder den Heizbehältnissen (28) und der Pumpe (27) sowie stromabwärts der Pumpe (27) jeweils mindestens eine Schnittstelle angeordnet ist, an der die Substitutionsleitung (25) getrennt werden kann, wobei die Schnittstellen derart miteinander kompatibel ausgebildet sind, dass das oder die Heizbehältnisse (28) alternativ auch stromabwärts der Pumpe (27) in die Substitutionsleitung (25) eingesetzt werden könnten.

5. Vorrichtung nach Anspruch 1, **dadurch gekennzeichnet, dass** die Substitutionsleitung im Bereich der stromabwärtigen Pumpe (31) eine Bypassleitung (32) aufweist, in der vorzugsweise ein Rückschlagventil (33) angeordnet ist, das einen Fluss nur in Richtung des extrakorporalen Blutkreislaufs (1) zulässt.

6. Vorrichtung nach Anspruch 1 Durchführung einer extrakorporalen Blutbehandlung, in der dem Patienten eine Substitutionsflüssigkeit verabreicht wird,
**dadurch gekennzeichnet,**
**dass** das stromaufwärtige Ende der Substitutionsleitung (25) mit einem Sterilfilter (20) versehen ist und in einen gasgefüllten Raum ragt.

7. Vorrichtung nach Anspruch 6, **dadurch gekennzeichnet, dass** die Vorrichtung ferner eine Steuereinheit und gegebenenfalls einen Sensor zur Messung der Fördermenge der Pumpe (27) aufweist, wobei die Steuereinheit mit der Pumpe (27) und gegebenenfalls mit dem Mengensensor in Verbindung steht und so ausgebildet ist, dass in einer Betriebsphase der Vorrichtung die Fördermenge der Pumpe (27) gemessen und die Pumpe (27) bei Erreichen einer bestimmten Gesamtfördermenge abgeschaltet wird.

8. Vorrichtung nach Anspruch 6 oder 7, **dadurch gekennzeichnet, dass** der extrakorporale Blutkreislauf (1) stromabwärts der Mündungsstelle der Substitutionsleitung (25) eine Tropfkammer (12) aufweist und dass die Vorrichtung ferner eine Steuereinheit und einen Sensor zur Messung des Füllstands der Tropfkammer (12) aufweist, wobei die Steuereinheit mit der Pumpe (27) und mit dem Füllstandsensor in Verbindung steht und so ausgebildet ist, dass in einer Betriebsphase der Füllstand der Tropfkammer (12) gemessen und die Pumpe (27) bei Erreichen eines bestimmten Mindestfüllstands abgeschaltet wird.

## Claims

1. An apparatus for carrying out an extracorporeal blood treatment in which a substitution fluid is administered to the patient, wherein the apparatus comprises an extracorporeal blood circuit (1) and a substitution line (25) opening into the extracorporeal blood circuit (1), and wherein the substitution line (25) has at least one heating container (28), wherein a pump (31) is arranged downstream of the heating container or containers (28) in the substitution line (25) for conveying substitution fluid into the extracorporeal blood circuit (1), and **characterized in that** a further pump (27) for conveying substitution fluid into the extracorporeal blood circuit (1) is arranged upstream of the heating container or containers (28) in the substitution line (25); and **in that** the apparatus furthermore has a control unit that is connected to the pumps (27, 31) and that is configured such that the upstream pump (27) is stopped and the downstream pump (31) is operated in an operating mode of the apparatus.

2. An apparatus in accordance with claim 1, **characterized in that** a clamp (34) which inhibits the flow of substitution fluid in the closed state is arranged upstream of the heating container or containers in the substitution line.

3. An apparatus in accordance with claim 2, **characterized in that** the apparatus furthermore has a control unit which is connected to the pump (27) and, optionally, furthermore to the clamp (34) and is configured such that the clamp is closed and the pump (27) is operated in an operating mode of the apparatus.

4. An apparatus in accordance with one of the preceding claims, **characterized in that** a respective at least one interface at which the substitution line (25) can be separated is arranged upstream of the heating container or containers (28), between the heating container or containers (28) and the pump (27) and downstream of the pump (27), with the interfaces being configured as compatible with one another such that the heating container or containers (28) can alternatively also be inserted into the substitution line (25) downstream of the pump (27).

5. An apparatus in accordance with claim 1, **characterized in that** the substitution line has a bypass line (32) in the region of the downstream pump (31), in which bypass line a check valve (33) is preferably arranged which only allows a flow in the direction of the extracorporeal blood circuit (1).

6. An apparatus in accordance with claim 1 for carrying out an extracorporeal blood treatment in which a substitution fluid is administered to the patient,
**characterized in that**
the upstream end of the substitution line (25) is provided with a sterile filter (20) and projects into a gas-filled space.

7. An apparatus in accordance with claim 6, **characterized in that** the apparatus furthermore has a control unit and, optionally, a sensor for measuring the conveying quantity of the pump (27), wherein the control unit is connected to the pump (27) and, optionally, to the quantity sensor and is configured such that the conveying quantity of the pump (27) is measured and the pump (27) is switched off on reaching a specific total conveying quantity in an operating phase of the apparatus.

8. An apparatus in accordance with claim 6 or claim 7, **characterized in that** the extracorporeal blood circuit (1) has a drop chamber (12) downstream of the opening point of the substitution line (25) and the apparatus furthermore has a control unit and a sensor for measuring the filling level of the drop chamber (12), with the control unit being connected to the pump (27) and to the filling level sensor and being configured such that the filling level of the drop chamber (12) is measured and the pump (27) is switched off on reaching a specific minimum filling level in an operating phase.

## Revendications

1. Dispositif pour réaliser un traitement extracorporel du sang, dans lequel un liquide de substitution est administré au patient, le dispositif comprenant un circuit sanguin extracorporel (1) et un conduit de substitution (25) débouchant dans le circuit sanguin extracorporel (1) et le conduit de substitution (25) comportant au moins un contenant chauffant (28), une pompe (31) pour l'acheminement de liquide de substitution dans le circuit sanguin extracorporel (1) étant disposée dans le conduit de substitution (25) en aval du ou des contenants chauffants (28) et **caractérisé en ce qu'**une autre pompe (27) pour l'acheminement de liquide de substitution dans le circuit sanguin extracorporel (1) est disposée dans le conduit de substitution (25) en amont du ou des contenants chauffants (28) et le dispositif comporte en outre une unité de commande, qui est reliée aux pompes (27, 31) et conçue de telle manière que, dans un mode de fonctionnement du dispositif, la pompe (27) côté amont est arrêtée et la pompe (31) côté aval est actionnée.

2. Dispositif selon la revendication 1, **caractérisé en ce qu'**une pince (34) est disposée dans le conduit de substitution en amont du ou des contenants chauffants, laquelle empêche, à l'état fermé, l'écoulement de solution de substitution.

3. Dispositif selon la revendication 2, **caractérisé en ce que** le dispositif comporte en outre une unité de commande, qui est reliée à la pompe (27) et le cas échéant en outre à la pince (34), et qui est conçue de telle manière que, dans un mode de fonctionnement du dispositif, la pince est fermée et la pompe (27) est actionnée.

4. Dispositif selon l'une des revendications précédentes, **caractérisé en ce qu'**au moins une interface est disposée respectivement en amont du ou des contenants chauffants (28), entre le ou les contenants chauffants (28) et la pompe (27) ainsi qu'en aval de la pompe (27), le conduit de substitution (25) pouvant être coupé au niveau de ladite interface, les interfaces étant réalisées de manière compatible entre elles de telle manière que le ou les contenants chauffants (28) peuvent être insérés dans le conduit de substitution (25) de manière alternative également en aval de la pompe (27).

5. Dispositif selon la revendication 1, **caractérisé en ce que** le conduit de substitution comporte, dans la zone de la pompe (31) côté aval, un conduit de dérivation (32), dans lequel est disposée de préférence une soupape de non-retour (33), qui permet un écoulement uniquement en direction du circuit sanguin extracorporel (1).

6. Dispositif selon la revendication 1 pour réaliser un traitement extracorporel du sang, dans lequel un liquide de substitution est administré au patient,
**caractérisé en ce que**
l'extrémité côté amont du conduit de substitution (25) est pourvue d'un filtre stérilisant (20) et dépasse dans un espace rempli de gaz.

7. Dispositif selon la revendication 6, **caractérisé en ce que** le dispositif comporte en outre une unité de commande et le cas échéant un capteur pour mesurer le débit de refoulement de la pompe (27), l'unité de commande étant reliée à la pompe (27) et le cas échéant au capteur de débit et étant conçue de telle manière que, dans un mode de fonctionnement du dispositif, le débit de refoulement de la pompe (27) est mesuré et la pompe (27) est désactivée lorsqu'un débit de refoulement total défini est atteint.

8. Dispositif selon la revendication 6 ou 7, **caractérisé en ce que** le circuit sanguin extracorporel (1) comporte une chambre compte-gouttes (12) en aval de l'embouchure du conduit de substitution (25) et **en ce que** le dispositif comporte en outre une unité de commande et un capteur pour mesurer le niveau de remplissage de la chambre compte-gouttes (12), l'unité de commande étant reliée à la pompe (27) et au capteur de niveau de remplissage et étant conçue de telle manière que, dans un mode de fonctionnement, le niveau de remplissage de la chambre compte-gouttes (12) est mesuré et la pompe (27) est désactivée lorsqu'un niveau de remplissage minimal défini est atteint.
